(19) **Europäisches Patentamt**

**European Patent Office**

**Office européen des brevets**

(11) Veröffentlichungsnummer : **0 173 251**
**B1**

(12) **EUROPÄISCHE PATENTSCHRIFT**

(45) Veröffentlichungstag der Patentschrift :
23.11.89

(21) Anmeldenummer : **85110543.7**

(22) Anmeldetag : **22.08.85**

(51) Int. Cl.⁴ : **C 07 H 21/00, C 07 J 43/00,
C 12 Q 1/68**

(54) Derivatisierte Nucleinsäure-Sequenz, Verfahren zu deren Herstellung sowie deren Verwendung zum Nachweis von Nucleinsäuren.

(30) Priorität : 28.08.84 DE 3431536

(43) Veröffentlichungstag der Anmeldung :
05.03.86 Patentblatt 86/10

(45) Bekanntmachung des Hinweises auf die Patenterteilung : 23.11.89 Patentblatt 89/47

(84) Benannte Vertragsstaaten :
AT BE CH DE FR GB IT LI LU NL SE

(56) Entgegenhaltungen :
EP-A- 0 138 357
EP-A- 0 158 758
WO-A-83 /022 77
WO-A-84 /032 85
GB-A- 2 019 408

(73) Patentinhaber : BOEHRINGER MANNHEIM GMBH
Sandhofer Strasse 116
D-6800 Mannheim 31 (DE)

(72) Erfinder : Graf, Hermann, Dr. rer. nat.
Johannes-Tanner-Strasse 2 a
D-8000 München 60 (DE)
Erfinder : Lenz, Helmut, Dr. rer. nat.
Waldschmidtstrasse 7
D-8132 Tutzing (DE)

## Beschreibung

Die Erfindung betrifft neue derivatisierte Nucleinsäure-Sequenzen, Verfahren zu deren Herstellung sowie deren Verwendung zum Nachweis und Charakterisieren von Nucleinsäuren mit komplementärer Nucleinsäure-Sequenz.

Nucleinsäure-Sequenzen werden üblicherweise durch Hybridisierung mit einer komplementären Nucleinsäure-Sequenz nachgewiesen, die mit einer nachweisbaren Gruppe markiert ist. Bisher erfolgt diese Markierung durch Einführung von Radioisotopen-tragenden Gruppen. Da das Arbeiten mit radioaktiven Substanzen und der Nachweis von Radioisotopen mit erheblichen Nachteilen (Sicherheitsvorkehrungen, apparativer Aufwand) verbunden ist, wurde verschiedentlich versucht, andere Markierungsmittel für den Hybridisierungstest zu verwenden.

Aus der deutschen Offenlegungsschrift 29 15 082 ist ein Verfahren zum Nachweis von Nucleinsäure-Sequenzen bekannt, bei dem diese mit einem Indikator hybridisiert wird, der eine komplementäre Nucleinsäure enthält und der durch Bindung an ein Enzym vor oder nach der Hybridisierungsreaktion chemisch modifiziert ist. Die Methode beruht im wesentlichen auf der seit langem bekannten Avidin-Biotin-Wechselwirkung. Hierzu wird die zum Nachweis einer Nucleinsäure-Sequenz eingesetzte komplementäre Nucleinsäure-Sequenz mit Avidin bzw. Biotin markiert. Nach dem Hybridisierungsschritt wird dann mit Biotin bzw. Avidin, das mit einem Enzym als Markierungsmittel substituiert ist, umgesetzt. Der Nachweis des Hybridisierungsproduktes erfolgt schließlich durch Bestimmung der enzymatischen Aktivität des Markerenzyms. Diese Methode hat den entscheidenden Nachteil, daß sie sehr störanfällig ist, weil Biotin in nahezu allen biologischen Materialien vorkommt.

In der WO 87/03285 ist ein Verfahren zur Synthese von mit Reportergruppen markierten Oligonukleotiden beschrieben. Dieses Verfahren benötigt als Ausgangsmaterial ein Oligonukleotid, in welches neben den natürlich vorkommenden Nukleinbasen modifizierte Basen eingebaut sind. Daher können nach diesem Verfahren nur künstlich synthetisierte Oligonukleotide markiert werden. Die Synthese solcher synthetischer Oligonukleotide ist aufwendig und daher kostenintensiv.

In der EP-A-0 158 758 ist ein Verfahren zur Herstellung von derivatisierten Nucleinsäuren beschrieben, in welchen ein direkt gebundener Jod-N-2-acetylaminofluorenrest als Marker dient.

In der WO/02277 ist ein Verfahren zur Kettenverlängerung von Nukleinsäuren beschrieben. Dieses Verfahren erfordert zunächst die mehrstufige Synthese von modifizierten Mononukleotidtriphosphaten und dann eine enzymatische Umsetzung mit einer Nucleinsäure. Diese Umsetzung ist insbesondere wegen der enzymatischen Reaktionsführung anfällig und aufwendig. Die Ausbeute der Markierung ist nicht hoch, was zur Folge hat, daß die Empfindlichkeit der mit solchen Nukleinsäuren durchgeführten Tests nicht hoch ist.

In der EP-A-0 138 357 ist eine Einzelstrang-DNA offenbart, an die über die Basen Adenin und Cytosin, nicht jedoch über die Nucleinbasen Guanin oder Thymin eine nachweisbare Gruppe gebunden ist. Als Brückenglied zwischen der DNA und der nachweisbaren Gruppe wird ein trifunktionelles Brückenglied vorgeschlagen, das über 2 Stickstoffatome der betreffenden Base mit der DNA und über eine Carboxylfunktion mit der nachweisbaren Gruppe verknüpft ist.

Aufgabe der vorliegenden Erfindung war es, die Antigen-Antikörper-Reaktion für den Hybridisierungstest auszunutzen. Die bisherigen Bemühungen hierzu sind offensichtlich daran gescheitert, daß es nicht möglich war, Nucleinsäure-Sequenzen in zuverlässiger Weise mit hinreichender Ausbeute mit einem geeigneten Antigen bzw. Antikörper zu markieren. Gelöst wird die Aufgabe durch Bereitstellen von derivatisierten Nucleinsäure-Sequenzen, die mit einem Partner einer immunologischen Reaktion markiert sind. Diese immunologische Methode hat den Vorteil, daß die erforderlichen Reagentien sehr stabil sind, daß die Reaktionsschritte in definierter Weise rasch und weitgehend vollständig ablaufen, daß die Umwelt wesentlich weniger belastet wird als bei den vorbekannten radioaktiven Methoden und daß die Kosten für die erfindungsgemäßen Teste deutlich niedriger liegen als bei den vorbekannten Methoden.

Gegenstand der Erfindung sind demnach derivatisierte Nucleinsäure-Sequenzen mit einem oder mehreren Strukturelementen der allgemeinen Formel I

$$X\text{—}A\text{—}M \qquad\qquad (I)$$

in der

X die Gruppe $>$N— oder $>$N—R—N' H—, wobei das N-Atom das Amin-Stickstoffatom einer Nucleinbase und R einen geradkettigen oder verzweigten niederen Alkylenrest, der durch Heteroatome unterbrochen sein kann, darstellen, wobei es sich bei den Nucleinbasen um die Basen Adenin, Guanin, Cytosin, Uracil, Thymin, Orotsäure, Xanthin, Hypoxanthin, 5-Hydroxymethylcytosin oder 5-Hydroxymethyl-uracil der Nucleinsäure handelt,

A ein bifunktionelles Brückenglied und

M ein Antigen, Hapten oder Antikörper bedeuten, sowie ein Verfahren zu deren Herstellung.

Als Partner einer immunologischen Reaktion M können alle möglichen Antigene und Haptene sowie die entsprechenden Antikörper eingesetzt werden. Der Ausdruck Antikörper steht sowohl für die vollständigen Antikörper als auch für Fragmente hiervon. Die Antikörper können sowohl in polyklonaler als auch in monoklonaler Form eingesetzt werden. In einer besonderen Ausführungsform wird die Nucleinsäure-Sequenz mit dem Antigen bzw. Hapten verknüpft, wobei die Haptene insbesondere Digoxin, $T_3$ und $T_4$ besonders bevorzugt sind. Als Haptene werden vorzugsweise hydrophile Haptene, beispielsweise Digoxin, eingesetzt, da sie weniger zu unspezifischen Bindungen mit einer Festphase neigen.

Als Brückenglied A eignet sich jede chemische Gruppierung, die geeignet ist, den Partner der immunologischen Reaktion derart mit der Nucleinsäure-Sequenz zu verbinden, daß die immunologische Aktivität des Partners nicht beeinflußt wird. Derartige Brückenglieder dürfen nicht zu hydrophob sein, sondern sollten eher hydrophile Eigenschaften aufweisen. Erfindungsgemäß geeignet sind vor allem bifunktionelle Gruppen, wie Dialdehyde, beispielsweise Glutardialdehyd, Dicarbonsäuren sowie deren Derivate, z. B. Diamide, Diester und Dianhydride, oder auch eine Kombination zweier oder mehrerer solcher Gruppen. Ferner kann das Brückenglied auch aus einer Peptidkette bestehen, die aus einem vollständigen Peptid, aus Peptid-Fragmenten oder aus Gruppierungen, die Peptidbindungen aufweisen, aufgebaut ist. Auch Oligosaccharide eignen sich vorzüglich als Brückenglieder. Wird beispielsweise Digoxin als Partner der immunologischen Reaktion verwendet, so stellt bereits der Trisaccharid-Rest des Digoxins eine geeignete Brücke dar.

Der geradkettige oder verzweigte niedere Alkylen-Rest in der Definition des Substituenten R weist vorzugsweise 1 bis 10, besonders bevorzugt 3 bis 5 Kohlenstoffatome auf. Ganz besonders bevorzugt ist die Propylen-Kette. Heteroatome, die den Alkylen-Rest R unterbrechen können, sind beispielsweise Sauerstoff-, Stickstoff- und Schwefelatome.

Der Partner einer immunologischen Reaktion M ist über das Brückenglied A mit einer Aminogruppe einer Nucleinbase verknüpft. Nucleinbasen sind die Basen Adenin, Guanin, Cytosin, Uracil, Thymin, Orotsäure, Xanthin, Hypoxanthin, 5-Hydroxymethylcytosin oder 5-Hydroxymethyluracil der Nucleinsäure. Cytosin und Guanin sind beispielsweise solche Nucleinbasen mit Aminogruppen, die sich leicht in erfindungsgemäßer Weise substituieren lassen.

Das Verfahren zur Herstellung der erfindungsgemäßen Nucleinsäure-Sequenzen der allgemeinen Formel I ist dadurch gekennzeichnet, daß man Nucleinsäure-Sequenzen, die eine oder mehrere Nucleinbasen mit der Gruppierung

$$X—H$$

enthalten, mit einer Verbindung der allgemeinen Formel II

$$Y—A—M \qquad (II)$$

in der
    Y einen reaktiven, mit einer Aminogruppe reagierenden Rest,
    A ein bifunktionelles Brückenglied und
    M einen Partner einer immunologischen Reaktion
darstellen, unter Abspaltung von HY umsetzt.

Verbindungen der allgemeinen Formel I, in denen X die Gruppe $>$N—R—N′ H— vorstellt, werden vorzugsweise durch ein Verfahren dargestellt, das dadurch gekennzeichnet ist, daß man Nucleinsäure-Sequenzen, die eine oder mehrere Nucleinbasen mit Aminogruppen enthalten, zunächst mit einem Polyamin der allgemeinen Formel III

$$H_2N—R—N′ H_2 \qquad (III),$$

in der R die oben genannte Bedeutung hat, in Gegenwart von Bisulfit-Anionen reagieren läßt und die erhaltenen Nucleinsäuren mit einem oder mehreren Strukturelementen der allgemeinen Formel IV

$$>N—R—N′ H_2$$
$$(IV),$$

in der R die oben angegebene Bedeutung hat, mit Verbindungen der allgemeinen Formel II

$$Y—A—M$$

in der
    Y einen reaktiven, mit einer Aminogruppe reagierenden Rest,
    A ein bifunktionelles Brückenglied und
    M einen Partner einer immunologischen Reaktion
darstellen, umsetzt.

Als reaktive Gruppe Y eignen sich ganz allgemein reaktive Gruppen, die mit einer Aminogruppe unter Ausbildung einer kovalenten Bindung zu reagieren vermögen. Besonders geeignet sind O-Succinimid-Gruppen.

Um die Derivatisierung der polymeren Nucleinsäure zu erreichen, wird die Nucleophilie der Aminogruppen an den Nucleinbasen, vorzugsweise Cytosin und Guanin, ausgenützt. Die Reaktionen erfolgen unter Bedingungen, die für derartige nucleophile Austauschreaktionen üblich sind. Der Austausch erfolgt statistisch an den Cytosin- und Guanin-Resten der eingesetzten Nucleinsäure.

Bei dieser Umsetzung reagieren die endständigen Amino-Gruppen mit größerer Ausbeute als die partiell aromatischen Gruppen am Cytosin-Ring. Diese Reaktionen sind vergleichbar mit den entsprechenden Umsetzungen an Mono-Nucleotiden, die in der Literatur zahlreich beschrieben sind. Auch die Reaktionsbedingungen werden in Analogie zu den beschriebenen Verfahren gewählt.

Die Nucleinsäure-Sequenzen, die eine oder mehrere Nucleinbasen mit Aminogruppen enthalten, sowie die modifizierten Nucleinsäuren mit einem oder mehreren Strukturelementen der allgemeinen Formel IV werden mit dem chemisch aktivierten Partner einer immunologischen Reaktion umgesetzt. Hierzu wird der Partner der immunologischen Reaktion mit dem Brückenglied A verknüpft und durch Einführen einer Gruppe Y chemisch aktiviert.

Die so hergestellten Nucleinsäure-Derivate eignen sich besonders gut zum Nachweis von Nucleinsäuren mit komplementären Nucleinsäure-Sequenzen. Derartige Teste sind vor allem von Bedeutung zum Nachweis von Viren und Bakterien oder auch bei der Untersuchung von Erbmaterialien. Hierzu wird eine Probe, die die nachzuweisenden, komplementären Nucleinsäuren bzw. Nucleinsäure-Sequenzen enthält, mit einem entsprechenden, erfindungsgemäßen Nucleinsäure-Derivat in Kontakt gebracht, das dabei entstehende Hybridisierungsprodukt wird mit dem markierten Partner der immunologischen Reaktion umgesetzt und die Markierung in bekannter Weise bestimmt.

Ein weiterer Gegenstand der Erfindung ist demgemäß die Verwendung der erfindungsgemäßen derivatisierten Nucleinsäure-Sequenzen zum Nachweis von Nucleinsäuren mit komplementärer Nucleinsäure-Sequenz, in dem eine Lösung mit der nachzuweisenden Nucleinsäure bzw. Nucleinsäure-Sequenz mit einer Lösung, die ein komplementäres erfindungsgemäß derivatisiertes Nucleinsäure-Derivat enthält, in Kontakt gebracht, nicht-hybridisierte Anteile von dem Nucleinsäure-Hybrid abgetrennt, die immunologische Reaktion des anderen markierten Partners der immunologischen Reaktion durchgeführt und die im erhaltenen Hybridisierungsprodukt vorhandene Markierung bestimmt wird.

Der eigentliche Hybridisierungsschritt zwischen der erfindungsgemäß derivatisierten Nucleinsäure-Sequenz und der komplementären Nucleinsäure in der Probe erfolgt nach bekannten Methoden. Besonders bevorzugt ist die Festphasen-Hybridisierung. Hierzu wird die nachzuweisende Nucleinsäure auf eine Festphase fixiert. Gegebenenfalls als Doppelstrang vorliegende Nucleinsäure muß in bekannter Weise zu Einzelsträngen aufgespalten werden. Als Festphase eignet sich im Prinzip jedes Nucleinsäurebindende Material. Üblicherweise werden Nitrozellulose-Membranen eingesetzt.

Für den Fixierungsschritt stehen bekannte Methoden zur Verfügung. Durch besondere Auswahl der Festphase, Blockierung mit Nichtimmunseren und gereinigten Fraktionen, Waschschritte mit Detergentien und ähnlicher an sich üblicher Verfahrensschritte, wird versucht, die unspezifische Bindung des anderen, markierten Partners der immunologischen Reaktion an die Festphase zu verhindern bzw. weitgehend einzuschränken.

Auch die Komplexbildung von Antigen und Antikörper wird nach bekannten Methoden durchgeführt. Prinzipiell sind hierzu alle möglichen, bekannten Verfahren anzuwenden.

Als Markierungsmittel können im Prinzip alle üblichen Markierungsmittel eingesetzt werden, beispielsweise Radioisotope, lichtemittierende Gruppen sowie Enzyme. Besonders bevorzugt ist die Verwendung von Enzymen als Markierung. Solche Indikatorenzyme sind bereits aus den immunologischen Bestimmungsverfahren bekannt. Besonders bevorzugt sind Peroxidase, alkalische Phosphatase und β-Galactosidase.

Die Messung der Markierung kann nach erfolgter Hybridisierung durchgeführt werden. Dem Fachmann stehen je nach Wahl des Markierungsmittels zahlreiche Methoden zur Verfügung. Werden Enzyme als Indikatoren verwendet, so werden zu deren Nachweis die üblichen Substrate eingesetzt.

Unter Verwendung der erfindungsgemäßen derivatisierten Nucleinsäure-Sequenz können in sehr empfindlicher Weise Nucleinsäuren nachgewiesen werden. Die Nachweisgrenzen liegen bei ungefähr 5 pg/Fleck für DNA bzw. bei ungefähr 200 pg/Fleck für RNA, wenn die Nachweisreaktion in Form der bekannten Tüpfel-Hybridisierung durchgeführt wird. Dies entspricht einer Empfindlichkeit von 20 Viroid-Molekülen pro Zelle, wenn beispielsweise infizierte Tomatenblätter untersucht werden.

Die Erfindung wird durch die folgenden Beispiele näher erläutert.

## Beispiel 1

Direkte Umsetzung einzelsträngiger DNA mit aktiviertem Ester

Die zu markierende DNA, beispielsweise 5 mg Plasmid-DNA, wird durch Spaltung mit einem Restriktionsenzym linearisiert und durch 10 minütiges Erhitzen auf 95 °C und anschließendem Abschrecken in Ethanol-Trockeneis denaturiert. Die DNA wird mit Ethanol gefällt und dann in 0,1 M Natriumhydrogencarbonat aufgenommen, wobei die Konzentration ca. 1mg/ml betragen sollte.

50 µl einer solchen DNA-Lösung werden mit 74 µl Dimethylformamid, 10 µl Digoxin-Glutaryl-O-Succinimid-Ester (20 µg pro µl in Dimethylformamid) versetzt. Dieses Reaktionsgemisch wird 2 Stunden bei 4 °C unter gelegentlichem Schütteln inkubiert. Danach wird 1/10 des volumens 20 %ige Kaliumacetatlösung zugegeben und mit Ethanol gefällt. Nicht umgesetzter Succinimidester wird durch eine zweite Ethanol-Fällung abgetrennt. Die markierte DNA wird zu einer Konzentration von 2 µg/µl in Pufferlösung (10 mMol Tris, 1 mMol EDTA, pH 8,0) aufgenommen. Es entsteht eine leicht trübe Suspension Die Ausbeute beträgt ca. 85 %, bezogen auf die eingesetzte DNA.

## Beispiel 2

Umsetzung von modifizierter DNA mit aktiviertem Ester

Um eine größere Markierungsdichte auf der DNA zu erreichen, werden die Aminogruppen am Cytosin nach dem Verfahren von Shapiro und Weisgras (Biochem. and Biophys. Research Comun. 40, (1970), S. 839-843) unter Bisulfit-Katalyse in aliphatische Aminogruppen umgewandelt.

5 mg linearisierte Plasmid-DNA wird durch 10 minütiges Erhitzen auf 95 °C und Abschrecken in Ethanol/Trockeneis denaturiert und sofort gegen frisch hergestellte Sulfitreaktionslösung bei Raumtemperatur 2 × 3 Stunden dialysiert (19,5 g 4-Morpholin-ethansulfonsäure (MES), 214,6 ml 37 %igen Natriumhydrogensulfit-Lösung, 249,9 ml Diaminopropan werden mit rauchender Salzsäure auf pH 6,2 eingestellt und auf 1 l aufgefüllt; anschließend werden 0,55 g Hydrochinon zugegeben). Das Dialysat wird bei 60 °C 90 Stunden inkubiert. Anschließend wird gegen 10 mMol Tris, pH 8,0, 1 mMol EDTA dialysiert und die DNA aus der Lösung durch Ethanol ausgefällt.

Die so modifizierte DNA wird wie in Beispiel 1 beschrieben mit dem aktivierten Digoxin-Glutaryl-O-Succinimid-Ester umgesetzt. Das Endprodukt ist eine leicht trübe Suspension. Die Ausbeute beträgt ca. 80 %, bezogen auf eingesetzte DNA.

## Beispiel 3

Nachweis von Plasmid-spezifischer DNA in einem Fragmentgemisch nach Spaltung des Plasmids mit einem Restriktionsenzym

### Variante a

Restriktionsfragmente von verschiedenen Plasmiden, die unterschiedlich große Anteile von Plasmid pBR322 enthalten, werden elektrophoretisch getrennt und auf Nitrozellulosefilter in bekannter Weise (J. Mol. Biol. 94 [1975] S. 51-69) aufgezogen. Das so erhaltene Präparat wird mit pBR322 DNA, die gemäß Beispiel 2 mit Digoxin markiert worden ist, hybridisiert. Es wird mit 10 % Normal-Schafserum versetzt und anschließend mit 10 mU/ml Peroxidase konjugiertem Antidigoxin-Antikörper inkubiert. Die Peroxidase-Aktivität wird schließlich mit Tetramethylbenzidin visualisiert.

Positiv reagierten jeweils nur diejenigen Restriktionsfragmente, die mit pBR322 komplementäre Sequenzen enthalten. Detektiert werden konnten Fragmente im Bereich 500 bis 4000 Basenpaare.

### Variante b

Die nach Variante a erhaltenen Restriktionsfragmente werden direkt im Gel (T. M. Shinnick et al. Nucleic Acid Research 2 [1975] S. 1911) mit pBR322 DNA, die nach Beispiel 2 mit Digoxin markiert worden ist, hybridisiert. Nach Zugabe von 10 % Normal-Schafserum wird mit 100 mU/ml Peroxidase-konjugiertem Antidigoxin-Antikörper inkubiert. Die Peroxidase-Aktivität wird mit Tetramethylbenzidin visualisiert.

Auf diese Weise konnten ebenfalls spezifisch zu pBR322 komplementäre Banden identifiziert werden. Die Nachweisempfindlichkeit liegt bei ca. 200 pg/Bande. Ein Nachweis war möglich im Bereich 1000 bis 8000 Basenpaare.

Die Dokumentation der Resultate kann dadurch erreicht werden, daß man das eingetrocknete Gel auf der Gelatine-Schicht des Peroxidase-Nachweissystems konserviert.

## Beispiel 4

Nachweis spezifischer DNA in Bakterienkolonien

Verschiedene Escherichia Coli-Stämme werden nach dem Verfahren von Taub und Thompson (Analyt. Biochem 126 [1982] S. 222-230) auf Filterpapier präpariert und mit pBR322 DNA, das nach Beispiel 2 mit Digoxin markiert worden ist, hybridisiert. Hybridisierungsbedingungen und Waschschritte wurden auf den Antikörpernachweis optimiert. Nach Inkubation mit Antidigoxin-Antikörper-Peroxidase-Konjugat (10 mU/ml) und Visualisierung auf Tetramethylbenzidingelatine konnten Bakterien, die pBR322 enthalten durch blaue Färbung von solchen, die kein Plasmid enthalten, unterschieden werden. Auch hier können die Ergebnisse durch Fotografieren bzw. Konservieren der angefärbten Filterpapiere dokumentiert werden.

## Beispiel 5

Nachweis von PSTV (Potato-Spindle-Tuber-Viroid)-Infektionen in Tomaten/Kartoffeln

Tomaten- bzw. Kartoffelblätter oder Kartoffelknollen bzw. -schalen werden in Puffer homogenisiert (Ultraturrax, 3 M Natriumchlorid, 300 mM Natriumcitrat, 30 mM Diethyldithiocarbamat, 0,1 % Triton ; Konzentration 0,1 g/ml). Nach Zentrifugation und Filtration wird mit DNase I und anschließend Proteinase K inkubiert und nach Verdünnung mit Ethanol gefällt. Das Ethanol-Präzipitat wird mit 2 M Lithiumchlorid aufgenommen und erneut mit Ethanol gefällt. Das Pellet wird im Extraktionspuffer ohne Diethylthiocarbamat und Triton aufgenommen und durch Filtration auf Nitrozellulose aufgebracht. Das so erhaltene Präparat wird mit Plasmid DNA pAV 401, das die komplette Sequenz des PSTV enthält und das nach Beispiel 2 mit Digoxin markiert worden ist, hybridisiert. Es wird 10 % Normalschafserum zugegeben und anschließend mit Antidigoxin-Antikörper-β-Galactosidase-Konjugat inkubiert. Die Visualisierung der mit PSTV-infizierten Proben erfolgt durch Inkubation mit dem β-Galactosidase-Substrat Chlorphenolrotgalactosid. Die Ergebnisse können mit Hilfe von Farbphotographien dokumentiert werden.

Auf die beschriebene Weise kann eine PSTV-Infektion in Tomaten und Kartoffeln mit einer Empfindlichkeit nachgewiesen werden, die dem beschriebenen Verfahren von Owens (Science 213 (1981) S. 670-672) mit $^{32}$P markierter Plasmid-DNA entspricht.

## Patentansprüche

1. Derivatisierte Nucleinsäure-Sequenz mit einem oder mehreren Strukturelementen der allgemeinen Formel

$$X\text{—}A\text{—}M \hspace{4cm} (I)$$

in der

X die Gruppe $>$N— oder $>$N—R—N′ H—, wobei das N-Atom das Amin-Stickstoffatom einer Nucleinbase und R einen geradkettigen oder verzweigten niederen Alkylenrest, der durch Heteroatome unterbrochen sein kann, darstellen, wobei es sich bei den Nucleinbasen um die Basen Adenin, Guanin, Cytosin, Uracil, Thymin, Orotsäure, Xanthin, Hypoxanthin, 5-Hydroxymethylcytosin oder 5-Hydroxymethyl-uracil der Nucleinsäure handelt,

A ein bifunktionelles Brückenglied und

M ein Antigen, Hapten oder Antikörper bedeuten.

2. Nucleinsäure-Sequenz gemäß Anspruch 1, dadurch gekennzeichnet, daß als Hapten Digoxin, $T_3$ oder $T_4$ verwendet wird.

3. Nucleinsäure-Sequenz gemäß einem der Ansprüche 1 oder 2, dadurch gekennzeichnet, daß als Brückenglied A ein Glutaryl-Rest verwendet wird.

4. Verfahren zur Herstellung einer Nucleinsäure-Sequenz mit einem oder mehreren Strukturelementen der allgemeinen Formel I

$$X\text{—}A\text{—}M \hspace{4cm} (I)$$

in der

X die Gruppe $>$N— oder $>$N—R—N′ H—, wobei das N-Atom das Amin-Stickstoffatom einer Nucleinbase und R einen geradkettigen oder verzweigten niederen Alkylenrest, der durch Heteroatome unterbrochen sein kann, darstellen, wobei es sich bei den Nucleinbasen um die Basen Adenin, Guanin, Cytosin, Uracil, Thymin, Orotsäure, Xanthin, Hypoxanthin, 5-Hydroxymethylcytosin oder 5-Hydroxymethyl-uracil der Nucleinsäure handelt,

A ein bifunktionelles Brückenglied und

M ein Antigen, Hapten oder Antikörper bedeuten, dadurch gekennzeichnet, daß man Nucleinsäure-Sequenzen, die eine oder mehrere Nucleinbasen mit der Gruppierung X-H anthalten, wobei es sich bei den Nucleinbasen um die Basen Adenin, Guanin, Cytosin, Uracil, Thymin, Orotsäure, Xanthin, Hypoxanthin, 5-Hydroxymethylcytosin oder 5-Hydroxymethyl-uracil der Nucleinsäure handelt, mit einer Verbindung der allgemeinen Formel II

$$Y\text{—}A\text{—}M \hspace{4cm} (II)$$

in der

Y einen reaktiven, mit einer Aminogruppe reagierenden Rest,

A ein bifunktionelles Brückenglied und

M ein Antigen, Hapten oder Antikörper darstellen, unter Abspaltung von HY umsetzt.

5. Verfahren zur Herstellung einer Nucleinsäure-Sequenz mit einem oder mehreren Strukturelementen der allgemeinen Formel I

$$X\text{---}A\text{---}M \tag{I}$$

in der

X die Gruppe $>N\text{---}R\text{---}N'\,H\text{---}$, wobei das N-Atom das Amin-Stickstoffatom einer Nucleinbase und R einen geradkettigen oder verzweigten niederen Alkylenrest, der durch Heteroatome unterbrochen sein kann, bedeuten, wobei es sich bei den Nucleinbasen um die Basen Adenin, Guanin, Cytosin, Uracil, Thymin, Orotsäure, Xanthin, Hypoxanthin, 5-Hydroxymethylcytosin oder 5-Hydroxymethyl-uracil der Nucleinsäure handelt,

A ein bifunktionelles Brückenglied und

M ein Antigen, Hapten oder Antikörper darstellen, dadurch gekennzeichnet, daß man Nucleinsäure-Sequenzen, die eine oder mehrere Nucleinbasen mit Aminogruppen enthalten, wobei es sich bei den Nucleinbasen um die Basen Adenin, Guanin, Cytosin, Uracil, Thymin, Orotsäure, Xanthin, Hypoxanthin, 5-Hydroxymethylcytosin oder 5-Hydroxymethyl-uracil der Nucleinsäure handelt, zunächst mit einem Polyamin der allgemeinen Formel III

$$H_2N\text{---}R\text{---}N'\,H_2 \tag{III}$$

in der R die oben angegebene Bedeutung hat, in Gegenwart von Bisulfit-Anionen reagieren läßt und die erhaltenen Nucleinsäuren mit einem oder mehreren Strukturelementen der allgemeinen Formel IV

$$>N\text{---}R\text{---}N'\,H_2 \tag{IV}$$

in der R die oben angegebene Bedeutung hat mit Verbindungen der allgemeinen Formel II

$$Y\text{---}A\text{---}M \tag{II}$$

in der

Y einen reaktiven mit einer Aminogruppe reagierenden Rest,

A ein bifunktionelles Brückenglied und

M ein Antigen, Hapten oder Antikörper darstellen umsetzt.

6. Verwendung von Nucleinsäure-Derivaten gemäß einem der Ansprüche 1 bis 3 zum Nachweis von Nucleinsäuren mit komplementärer Nucleinsäure-Sequenz.

7. Verfahren zur Bestimmung einer Nucleinsäure bzw. einer Nucleinsäure-Sequenz durch Hybridisierung mit einer zur gesuchten Sequenz komplementären Nucleinsäure-Sequenz, die eine Markierung trägt, und Nachweis der Markierung, dadurch gekennzeichnet, daß als zur gesuchten Sequenz komplementäre Nucleinsäure-Sequenz eine mit einem Partner einer immunologischen Reaktion derivatisierte Nucleinsäure-Sequenz gemäß einem der Ansprüche 1 bis 3 eingesetzt und das Hybridisierungsprodukt mit dem anderen Partner der immunologischen Reaktion, der eine nachweisbare Markierung trägt, umgesetzt wird.

8. Verfahren gemäß Anspruch 7, dadurch gekennzeichnet, daß die nachweisbare Markierung eine Enzymmarkierung vorstellt.

9. Verfahren gemäß Anspruch 8, dadurch gekennzeichnet, daß als Markierungsenzym Peroxidase, alkalische Phosphatase oder β-Galactosidase eingesetzt wird.

## Claims

1. Derivatised nucleic acid sequence with one or more structural elements of the general formula:

$$X\text{---}A\text{---}M \tag{I}$$

in which

X signifies the group $>N\text{---}$ or $>N\text{---}R\text{---}N'H\text{---}$, whereby the N-atom represents the amine nitrogen atom of a nuclein base and R a straight-chained or branched lower alkylene radical which can be interrupted by heteroatoms, whereby in the case of the nuclein bases, it is a question of the bases adenine, guanine, cytosine, uracil, thymine, orotic acid, xanthine, hypoxanthine, 5-hydroxymethylcytosine or 5-hydroxymethyluracil of the nucleic acid,

A a bifunctional bridge member and

M an antigen, hapten or antibody.

2. Nucleic acid sequence according to claim 1, characterised in that digoxin, $T_3$ or $T_4$ is used as hapten.

3. Nucleic acid sequence according to one of claims 1 or 2, characterised in that a glutaryl radical is used as bridge member A.

7

4. Process for the preparation of a nucleic acid sequence with one or more structural elements of the general formula I

$$X—A—M \qquad (I)$$

in which

X signifies the group $>N—$ or $>N—R—N'H—$, whereby the N-atom represents the amine nitrogen atom of a nuclein base and R a straight-chained or branched lower alkylene radical which can be interrupted by heteroatoms, whereby in the case of the nuclein bases, it is a question of the bases adenine, guanine, cytosine, uracil, thymine, orotic acid, xanthine, hypoxanthine, 5-hydroxymethylcytosine or 5-hydroxymethyluracil of the nucleic acid,

A a bifunctional bridge member and

M an antigen, hapten or antibody, characterised in that one reacts nucleic acid sequences which contain one or more nuclein bases with the grouping X—H, whereby in the case of the nuclein bases, it is a question of the bases adenine, guanine, cytosine, uracil, thymine, orotic acid, xanthine, hypoxanthine, 5-hydroxymethylcytosine or 5-hydroxymethyluracil of the nucleic acid, with a compound of the general formula :

$$Y—A—M \qquad (II)$$

in which

Y represents a reactive residue reacting with an amino group,

A a bifunctional bridge member and

M an antigen, hapten or antibody, with splitting off of HY.

5. Process for the preparation of a nucleic acid sequence with one or more structural elements of the general formula I

$$X—A—M \qquad (I)$$

in which

X represents the group $>N—R—N'H—$, whereby the N-atom signifies the amine nitrogen atom of a nuclein base and R a straight-chained or branched lower alkylene radical which can be interrupted by heteroatoms, whereby in the case of nuclein bases, it is a question of the bases adenine, guanine, cytosine, uracil, thymine, orotic acid, xanthine, hypoxanthine, 5-hydroxymethylcytosine or 5-hydroxymethyluracil of the nucleic acid,

A a bifunctional bridge member and

M an antigen, hapten or antibody, characterised in that one first allows nucleic acid sequences which contain one or more nuclein bases with amino groups, whereby in the case of the nuclein bases, it is a question of the bases adenine, guanine, cytosine, uracil, thymine, orotic acid, xanthine, hypoxanthine, 5-hydroxymethylcytosine or 5-hydroxymethyluracil of the nucleic acid, to react with a polyamine of the general formula III

$$H_2N—R—N'H_2 \qquad (III)$$

in which R has the above-given meaning, in the presence of bisulphite anions and reacts the nucleic acids obtained with one or more structural elements of the general formula IV

$$>N—R—N'H_2 \qquad (IV)$$

in which R has the above-given meaning, with compounds of the general formula II

$$Y—A—M \qquad (II)$$

in which

Y is a reactive residue reacting with an amino group,

A a bifunctional bridge member and

M an antigen, hapten or antibody.

6. Use of nucleic acid derivatives according to one of claims 1 to 3 for the detection of nucleic acids with complementary nucleic acid sequence.

7. Process for the determination of a nucleic acid or of a nucleic acid sequence by hybridisation with a nucleic acid sequence complementary to the sought sequence, which carries a labelling, and detection of the labelling, characterised in that, as nucleic acid sequence complementary to the sought sequence,

8

there is used a nucleic acid sequence derivatised with a partner of an immunological reaction according to one of claims 1 to 3 and the hybridisation product is reacted with the other partner of the immunological reaction which carries a detectable labelling.

8. Process according to claim 7, characterised in that the detectable labelling represents an enzyme labelling.

9. Process according to claim 8, characterised in that, as labelling, there is used peroxidase, alkaline phosphatase or β-galactosidase.

## Revendications

1. Séquence de dérivé d'acide nucléique comprenant un ou plusieurs éléments de structure répondant à la formule générale I

$$X—A—M \qquad (I)$$

où

X représente le groupe $>N$ ou $>N—R—N'H—$, l'atome N c'est-à-dire l'atome d'azote de l'amine représentant une base nucléique et R un groupe alkylène inférieur, à chaîne droite ou ramifiée, qui peut être interrompue par un hétéroatome, les bases nucléiques pouvant être les bases adénine, guanine, cytosine, uracile, thymine, acide orotique, xanthine, hypoxanthine, 5-hydroxyméthylcytosine ou 5-hydroxyméthylcytosine de l'acide nucléique,
A représente un chaînon de liaison bifonctionnel et
M représente un antigène, un haptène ou un anticorps.

2. Séquence de dérivé d'acide nucléique selon la revendication 1 caractérisée en ce que comme haptène, on utilise la digoxine, $T_3$ ou $T_4$.

3. Séquence de dérivé d'acide nucléique, selon la revendication 1 ou 2, caractérisée en ce que comme chaînon de liaison A, on utilise un reste glutaryle.

4. Procédé pour la fabrication d'une séquence de dérivé d'acide nucléique comprenant un ou plusieurs éléments de structure répondant à la formule générale I

$$X—A—M \qquad (I)$$

où

X représente le groupe $>N$ ou $>N—R—N'H—$, l'atome N c'est-à-dire l'atome d'azote de l'amine représentant une base nucléique et R un groupe alkylène inférieur, à chaîne droite ou ramifiée, qui peut être interrompue par un hétéroatome, les bases nucléiques pouvant être les bases adénine, guanine, cytosine, uracile, thymine, acide orotique, xanthine, hypoxanthine, 5-hydroxyméthylcytosine ou 5-hydroxyméthylcytosine de l'acide nucléique,
A représente un chaînon de liaison bifonctionnel et
M représente un antigène, un haptène ou un anticorps,
caractérisé en ce que l'on fait réagir des séquences d'acides nucléiques, qui contiennent une ou plusieurs bases nucléiques contenant un groupement X—H, les bases nucléiques pouvant être les bases adénine, guanine, cytosine, uracile, thymine, acide orotique, xanthine, hypoxanthine, 5-hydroxyméthylcytosine ou 5-hydroxyméthylcytosine de l'acide nucléique, avec un composé de formule générale II

$$Y—A—M \qquad (II)$$

où
Y représente un groupe réactif, réagissant avec un groupe amino,
A un chaînon de liaison bifonctionnel et
M un antigène, un haptène ou un anticorps,
avec séparation de YH.

5. Procédé pour la fabrication d'une séquence de dérivé d'acide nucléique comprenant un ou plusieurs éléments de structure répondant à la formule générale I

$$X—A—M \qquad (I)$$

où

X représente le groupe $>N$ ou $>N—R—N'H—$, l'atome N c'est-à-dire l'atome d'azote de l'amine représentant une base nucléique et R un groupe alkylène inférieur, à chaîne droite ou ramifiée, qui peut être interrompue par un hétéroatome, les bases nucléiques pouvant être les bases adénine, guanine, cytosine, uracile, thymine, acide orotique, xanthine, hypoxanthine, 5-hydroxyméthylcytosine ou 5-hydroxyméthylcytosine de l'acide nucléique,

A représente un chaînon de liaison bifonctionnel et

M représente un antigène, un haptène ou un anticorps, caractérisé en ce que l'on fait réagir des séquences d'acides nucléiques, qui contiennent une ou plusieurs bases nucléiques contenant un groupement X-H, les bases nucléiques pouvant être les bases adénine, guanine, cytosine, uracile, thymine, acide orotique, xanthine, hypoxanthine, 5-hydroxyméthylcytosine ou 5-hydroxyméthylcytosine de l'acide nucléique, d'abord avec une polyamine de formule générale III

$$H_2N—R—N'H_2 \qquad (III)$$

où R a la signification mentionnée ci-dessus, en présence d'anions bisulfite, et que l'on fait réagir les acides nucléiques avec un ou plusieurs éléments structurels de formule générale IV

$$>N—R—N'H_2 \qquad (IV)$$

où R a la signification mentionnée ci-dessus, avec des composés de formule II

$$Y—A—M \qquad (II)$$

où

Y représente un groupe réactif, réagissant avec un groupe amino,
A un chaînon de liaison bifonctionnel et
M un antigène, un haptène ou un anticorps

6. Utilisation de dérivé d'acide nucléique selon l'une des revendications 1 à 3, pour la détermination d'acides nucléiques avec des séquences d'acide nucléiques complémentaires.

7. Procédé pour la détermination d'un acide nucléique ou d'une séquence d'acide nucléique par hybridation avec une séquence d'acide nucléique complémentaire de la séquence recherchée, qui porte un marquage, et détection du marquage, caractérisé en ce que comme séquence d'acide nucléique complémentaire de la séquence recherchée, on utilise une séquence de dérivé d'acide nucléique dérivée avec un partenaire d'une réaction immunologique selon l'une des réactions 1 à 3, et que l'on fait réagir le produit d'hybridation avec l'autre partenaire de la réaction immunologique, qui porte un marquage détectable.

8. Procédé selon la revendication 7, caractérisé en ce que le marquage détectable est un marquage enzymatique.

9. Procédé selon la revendication 7, caractérisé en ce que comme enzyme de marquage, on utilise la peroxydase, la phosphatase alcaline ou la β-galactosidase.